(19) **Europäisches Patentamt / European Patent Office / Office européen des brevets**

(11) **EP 4 616 722 A1**

(12) **EUROPEAN PATENT APPLICATION**
published in accordance with Art. 153(4) EPC

(43) Date of publication:
**17.09.2025  Bulletin 2025/38**

(21) Application number: **23888638.6**

(22) Date of filing: **06.11.2023**

(51) International Patent Classification (IPC):
**A23L 5/00** (2016.01)     **A23L 2/00** (2006.01)
**A23L 2/52** (2006.01)     **A23L 2/66** (2006.01)
**A23L 7/104** (2016.01)     **C12N 9/10** (2006.01)
**C12N 9/28** (2006.01)     **C12P 19/14** (2006.01)
**C12P 19/18** (2006.01)

(52) Cooperative Patent Classification (CPC):
**A23L 2/00; A23L 2/52; A23L 2/66; A23L 5/00;
A23L 7/104; C12N 9/10; C12N 9/2414;
C12P 19/14; C12P 19/18**

(86) International application number:
**PCT/JP2023/039815**

(87) International publication number:
**WO 2024/101290 (16.05.2024 Gazette 2024/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.11.2022  JP 2022178278**

(71) Applicant: **Amano Enzyme Inc.**
**Nagoya-shi**
**Aichi 460-8630 (JP)**

(72) Inventors:
• **TABUSE Shiho**
  **Kakamigahara-shi, Gifu 509-0109 (JP)**
• **TAKAYAMA Hiroyuki**
  **Kakamigahara-shi, Gifu 509-0109 (JP)**

(74) Representative: **Mullholland, Lewis Paul**
**WP Thompson**
**138 Fetter Lane**
**London EC4A 1BT (GB)**

(54) **METHOD FOR PRODUCING PLANT-BASED FOODS AND BEVERAGES**

(57)    To provide a technique for sufficiently promoting liquefaction during the production of plant-based foods and beverages and increasing the cyclodextrin content in the produced plant-based foods and beverages.

The technique provides a method for producing plant-based foods and beverages, the method including: a cyclodextrin-producing enzyme action step in which a cyclodextrin-producing enzyme is allowed to act on a plant-based raw material; and a cyclodextrin-producing enzyme and $\alpha$-amylase action step in which a cyclodextrin-producing enzyme and $\alpha$-amylase are allowed to act thereon after the cyclodextrin-producing enzyme action step.

**Description**

Technical Field

The present technique relates to a method for producing a plant-based food and beverage. More specifically, the present technique relates to a method for producing a plant-based food and beverage, a method for increasing the cyclodextrin content in a plant-based food and beverage, an agent for increasing the cyclodextrin content in a plant-based food and beverage, and a plant-based food and beverage.

Background Art

In recent years, plant-based foods and beverages have been attracting attention due to the growing health consciousness. For example, an oat beverage is prepared by suspending an oat material in an aqueous medium and decomposing the starch of the oat material using one or more types of amylases. The amylase used in this case is $\beta$-amylase or a mixture of $\alpha$-amylase and $\beta$-amylase. The oat beverage prepared by this method has a moderate sweetness due to the production of maltose by the action of $\beta$-amylase (Patent Literature 1).

Here, the enzymes used in the present technique will be described. Cyclodextrin-producing enzymes are enzymes acting on starch to produce $\alpha$-, $\beta$-, and $\gamma$-cyclodextrins. It is known that cyclodextrin-producing enzymes can act on starch to produce cyclodextrins without liquefying starch granules (Patent Literature 2).

Citation List

Patent Literature

Patent Literature 1: Japanese Patent Application Publication No. 2018-075029
Patent Literature 2: Japanese Patent Application Publication No. hei-09-262091

Summary of Invention

Technical Problem

As mentioned above, plant-based foods and beverages, which have been attracting attention in recent years, have a flavor derived from plant-based raw materials, and it is desirable to reduce the unpleasant flavor. In order to reduce the unpleasant flavor, it is effective to add cyclodextrin, which has a masking effect, to the plant-based foods and beverages. This cyclodextrin can be produced from starch by a cyclodextrin-producing enzyme.

On the other hand, when producing plant-based foods and beverages, the liquefaction of plant-based raw materials can be sufficiently promoted by allowing $\alpha$-amylase to act on the plant-based raw materials. However, as shown in the Examples described later, it has been found that the cyclodextrin content in the plant-based foods and beverages is reduced when $\alpha$-amylase and a cyclodextrin-producing enzyme are allowed to act on the plant-based raw materials compared to a case where the cyclodextrin-producing enzyme is used alone.

Therefore, the main objective of the present technique is to provide a technique that sufficiently advances liquefaction during the production of plant-based foods and beverages and increases the cyclodextrin content in the produced plant-based foods and beverages.

Solution to Problem

The inventors of the present application have conducted intensive research into techniques for increasing the cyclodextrin content in plant-based foods and beverages, and as a result have discovered that by allowing a cyclodextrin-producing enzyme to act on the plant-based raw materials that are the raw materials for plant-based foods and beverages, and then allowing $\alpha$-amylase to act thereon while continuing the treatment with the cyclodextrin-producing enzyme, it is possible to suppress the decrease in the cyclodextrin content in the plant-based foods and beverages caused by $\alpha$-amylase while still advancing sufficient liquefaction during the production of the plant-based foods and beverages, and ultimately to successfully increase the cyclodextrin content in the plant-based foods and beverages, leading to completion of the present technique.

That is, the present technique first provides a method for producing a plant-based food and beverage, including

a cyclodextrin-producing enzyme action step in which a cyclodextrin-producing enzyme is allowed to act on a plant-based raw material; and

a cyclodextrin-producing enzyme and α-amylase action step in which a cyclodextrin-producing enzyme and α-amylase are allowed to act thereon after the cyclodextrin-producing enzyme action step.

The present technique also provides a method for increasing the cyclodextrin content in a plant-based food and beverage, including

a cyclodextrin-producing enzyme action step in which a cyclodextrin-producing enzyme is allowed to act on a plant-based raw material; and

a cyclodextrin-producing enzyme and α-amylase action step in which a cyclodextrin-producing enzyme and α-amylase are allowed to act thereon after the cyclodextrin-producing enzyme action step.

The present technique further provides an agent for increasing the cyclodextrin content in a plant-based food and beverage, including

a first agent containing a cyclodextrin-producing enzyme; and
a second agent containing α-amylase.

The present technique also provides a plant-based food and beverage in which the agent for increasing the cyclodextrin content according to the present technique is used.

Advantageous Effects of Invention

According to the present technique, it is possible to sufficiently advance liquefaction during the production of a plant-based food and beverage, while increasing the cyclodextrin content in the produced plant-based food and beverage.

Description of Embodiments

A preferred embodiment for carrying out the present technique will be described below. Note that the embodiment described below shows an example of a typical embodiment of the present technique, and the scope of the present technique is not to be interpreted narrowly by this.

1. Method for producing plant-based food and beverage, and method for increasing cyclodextrin content in plant-based food and beverage

The method for producing a plant-based food and beverage and the method for increasing the cyclodextrin content according to the present technique are methods including a step of allowing a cyclodextrin-producing enzyme to act on a plant-based raw material (hereinafter also referred to as a "cyclodextrin-producing enzyme action step"), and a step of allowing a cyclodextrin-producing enzyme and α-amylase to act thereon (hereinafter also referred to as a "cyclodextrin-producing enzyme and α-amylase action step"). In addition, depending on the type of plant-based food and beverage, it is also possible to carry out general food production steps before, after, or simultaneously with each step, as long as the effects of the present technique are not impaired. Furthermore, in the method for producing a plant-based food and beverage and the method for increasing the cyclodextrin content, a recovery step of recovering the produced plant-based food and beverage or the plant-based food and beverage with an increased cyclodextrin content can be further carried out.

In the method for producing a plant-based food and beverage and the method for increasing the cyclodextrin content according to the present technique, it is preferable not to carry out a step of allowing α-amylase to act on a plant-based raw material (hereinafter also referred to as "α-amylase action step") before the cyclodextrin-producing enzyme action step. However, it is possible to carry out an α-amylase action step before the cyclodextrin-producing enzyme action step, so long as it does not affect the amount of cyclodextrin produced. The raw materials used and each step will be described in detail below.

(1) Plant-based raw material

The plant-based raw materials that can be used in the present technique are not particularly limited in origin, type, etc., as long as they do not impair the effects of the present technique, and can be freely selected according to the intended plant-based food and beverage. For example, beans such as soy beans, green peas, lentils, chickpeas, black beans, broad beans, mung beans, lupine beans, kidney beans, etc.; grains such as wheat, barley, oats, rice, rye, buckwheat, barnyard millet, millet, teff, etc.; nuts such as almonds, coconuts, peanuts, cashew nuts, hazelnuts, pecan nuts, macadamia nuts, pistachios, walnuts, Brazil nuts, pili nuts, chestnuts, sesame seeds, pine nuts, etc.; hemp seeds (industrial hemp), chia seeds, quinoa, amaranthus, canary seeds, flaxseeds, etc., are mentioned. In the present technique, one type of them may be used alone, or two or more types may be used in combination. Among these raw materials, preferred are cereals, and more preferred are oats.

In the present technique, the properties of a plant-based raw material when subjected to various enzyme treatments are not particularly limited as long as they do not impair the effects of the present technique, but preferred forms include liquid, slurry, and paste.

The plant-based raw material that can be used in the present technique contains carbohydrates. The content of the plant-based carbohydrates contained in the plant-based raw material (based on the weight of the plant-based raw material in a dry state) is not particularly limited, but may be, for example, 20% by weight or more. Preferably, it is 30% by weight or more, more preferably 40% by weight or more, and even more preferably 50% by weight or more. The upper limit of the content range is not particularly limited, but may be, for example, 90% by weight or less, 80% by weight or less, or 70% by weight or less.

In the present technique, it is preferable to use a plant-based raw material that has not been treated with $\alpha$-amylase when carrying out a cyclodextrin-producing enzyme action step described below. When liquefying starch contained in a plant-based raw material, it is possible to increase the amount of cyclodextrin in the plant-based food and beverage after liquefaction by treating it with a cyclodextrin-producing enzyme before the $\alpha$-amylase treatment and then treating it with $\alpha$-amylase and the cyclodextrin-producing enzyme.

(2) Cyclodextrin-producing enzyme action step

The cyclodextrin-producing enzyme action step is a step in which a cyclodextrin-producing enzyme is allowed to act on a plant-based raw material. In the cyclodextrin-producing enzyme action step, it is preferable to allow the cyclodextrin-producing enzyme to act singly on the plant-based raw material, but one or more types of other enzymes that can be used in the production of plant-based foods and beverages can be selected and used in combination as long as they do not affect the amount of cyclodextrin produced.

The cyclodextrin-producing enzyme that can be used in the present technique is an enzyme that has an activity of producing cyclodextrin having a degree of polymerization of 6 to 8 using a polysaccharide having an $\alpha$-1,4-bonded glucose structure, such as starch, as a substrate. The cyclodextrin-producing enzyme that can be used in the present technique may be an enzyme that further has another function, so long as the activity of producing cyclodextrin is its main activity.

In this technique, by allowing a cyclodextrin-producing enzyme to act on a plant-based raw material, it is possible to liquefy starch in the plant-based raw material and increase the amount of cyclodextrin in the produced plant-based food and beverage. The cyclodextrin in the produced plant-based food and beverage can mask off-flavors.

The origin of the cyclodextrin-producing enzyme that can be used in the present technique is not particularly limited, and examples thereof include cyclodextrin-producing enzymes derived from the genus Bacillus, such as Bacillus stearothermophilus, Bacillus megaterium, Bacillus circulans, Bacillus macerans, Bacillus ohbensis, and Bacillus clarkii; the genus Geobacillus, such as Geobacillus stearothermophilus; the genus Paenibacillus, such as Paenibacillus macerans; the genus Klebsiella, such as Klebsiella pneumoniae; the genus Thremoanaerobacter; and the genus Brevibacterium. One type of these cyclodextrin-producing enzymes may be used alone, or several types may be used in combination. Among them, from the viewpoint of improving the flavor of a plant-based raw material, the cyclodextrin-producing enzyme is preferably cyclodextrin glucanotransferase derived from Paenibacillus and/or cyclodextrin glucanotransferase derived from Anoxybacillus caldiproteolyticus (including the one previously called Geobacillus stearothermophilus), and more preferably cyclodextrin glucanotransferase derived from Paenibacillus macerans and/or cyclodextrin glucanotransferase derived from Anoxybacillus caldiproteolyticus (including the one previously called Geobacillus stearothermophilus).

Here, "cyclodextrin-producing enzyme derived from Paenibacillus macerans" and/or "cyclodextrin-producing enzyme derived from Anoxybacillus caldiproteolyticus (including the one previously called Geobacillus stearothermophilus)" means a cyclodextrin-producing enzyme produced by a microorganism (whether a wild-type strain or a mutant strain) classified as Paenibacillus macerans and/or Anoxybacillus caldiproteolyticus (including the one previously called Geobacillus stearothermophilus), or a cyclodextrin-producing enzyme obtained by genetic engineering approach using a cyclodextrin-producing enzyme gene. Therefore, recombinants produced by a host microorganism into which a cyclodextrin-producing enzyme gene (or a modified version of said gene) obtained from Paenibacillus macerans and/or Anoxybacillus caldiproteolyticus (including the one previously called Geobacillus stearothermophilus) has been introduced also fall under the category of "cyclodextrin-producing enzyme derived from Paenibacillus macerans" and/or "cyclodextrin-producing enzyme derived from Anoxybacillus caldiproteolyticus (including the one previously called Geobacillus stearothermophilus)".

The cyclodextrin-producing enzyme used in the present technique can be prepared from a culture solution of a microorganism from which the above-mentioned cyclodextrin-producing enzyme is derived. Specific preparation methods include a method of recovering the cyclodextrin-producing enzyme from the culture solution or microbial bodies of the above-mentioned microorganism. For example, when a cyclodextrin-producing enzyme-secreting microorganism is used, the microbial bodies can be recovered from the culture solution in advance by filtration, centrifugation, or the like as necessary, and the enzyme can then be separated and/or purified. When a cyclodextrin-producing enzyme non-secreting microorganism is used, the microbial bodies can be recovered from the culture solution in advance, if necessary, and then the microbial bodies can be crushed by pressure treatment, ultrasonic treatment or the like to expose the enzyme, and the enzyme can then be separated and/or purified. As the method for separating and/or purifying the enzyme, a known protein separation and/or purification method can be used without any particular limitation, and examples of such methods include centrifugation, UF concentration, salting out, and various chromatography methods using ion exchange resins, etc. The separated and/or purified enzyme can be powdered by a drying method such as freeze-drying or drying under reduced pressure, or can be powdered by using an appropriate excipient and/or drying aid in the drying method. The separated and/or purified enzyme can also be liquefied by adding an appropriate additive and sterilizing by filtration.

In the present technique, a commercially available product can be used as the cyclodextrin-producing enzyme, and preferred examples of the commercially available product include cyclodextrin glucanotransferase derived from Paenibacillus macerans manufactured by Amano Enzyme Inc., cyclodextrin glucanotransferase derived from Anoxybacillus caldiproteolyticus (previously classified as Geobacillus stearothermophilus) manufactured by Amano Enzyme Inc., and the like.

The amount of the cyclodextrin-producing enzyme added in the cyclodextrin-producing enzyme action step can be freely set as long as it does not impair the effects of the present technique. The lower limit of the amount of cyclodextrin-producing enzyme added can be set to, for example, 0.01 U or more per 1 g of a plant-based raw material, and from the viewpoint of further increasing the cyclodextrin content in the plant-based food and beverage, it can be set to preferably 0.03 U or more, more preferably 0.1 U or more, even more preferably 0.3 U or more, and even more preferably 1 U or more.

The lower limit of the amount of the cyclodextrin-producing enzyme added can be set, for example, to 0.02 U or more per 1 g of a polysaccharide (preferably starch) used in the production of a plant-based food and beverage, and from the viewpoint of further increasing the cyclodextrin content in the plant-based food and beverage, it can be set to preferably 0.05 U or more, more preferably 0.5 U or more, even more preferably 2 U or more, and even more preferably 5 U or more.

The upper limit of the amount of the cyclodextrin-producing enzyme added is not particularly limited as long as it does not impair the effects of the present technique, but can be set to, for example, 200 U or less, 100 U or less, 50 U or less, 20 U or less, or 10 U or less per 1 g of a plant-based raw material.

The upper limit of the amount of the cyclodextrin-producing enzyme added can be set to, for example, 500 U or less, 300 U or less, 150 U or less, 80 U or less, 60 U or less, or 40 U or less per 1 g of a polysaccharide (preferably starch) used in the production of a plant-based food and beverage.

In the present technique, the activity of a cyclodextrin-producing enzyme is a value measured by the measurement method described in the Examples below.

Various conditions for the cyclodextrin-producing enzyme action step can be freely set as long as they do not impair the effects of the present technique. For example, the pH, temperature, action time, etc. can be set according to the physicochemical properties of the cyclodextrin-producing enzyme used, such as the optimum pH, stable pH range, optimum temperature, and temperature stability. The pH can be set to, for example, pH 5.0 to 8.0, preferably pH 5.5 to 7.5, and more preferably pH 5.5 to 7.0. The temperature can be set to, for example, 30°C to 90°C, preferably 40°C to 85°C, and more preferably 50°C to 80°C. The action time can be set to, for example, 10 minutes to 24 hours, preferably 20 minutes to 12 hours, and more preferably 30 minutes to 240 minutes as the total reaction time, and the reaction time for the treatment with the cyclodextrin-producing enzyme alone can be set to, for example, 5 minutes to 12 hours, preferably 10 minutes to 6 hours, and more preferably 20 minutes to 150 minutes. The optimal reaction conditions can be determined through preliminary experiments.

(3) Cyclodextrin-producing enzyme and α-amylase action step

The cyclodextrin-producing enzyme and α-amylase action step is a step in which the cyclodextrin-producing enzyme and α-amylase are allowed to act on the plant-based raw material that has been subjected to the cyclodextrin-producing enzyme action step. In this technique, the cyclodextrin-producing enzyme and α-amylase are allowed to act on the plant-based raw material that has been treated with the cyclodextrin-producing enzyme, thereby making it possible to increase the cyclodextrin content in the produced plant-based food and beverage.

In this technique, the cyclodextrin-producing enzyme and α-amylase can be added to perform the cyclodextrin-producing enzyme and α-amylase action step, after the cyclodextrin-producing enzyme action step, but it is preferable to proceed with the cyclodextrin-producing enzyme and α-amylase action step by adding α-amylase without inactivating the cyclodextrin-producing enzyme after the cyclodextrin-producing enzyme action step. By adding α-amylase while continuing the cyclodextrin-producing enzyme treatment of a plant-based raw material, the addition of each enzyme can be completed in one go, simplifying the production process and improving workability.

The α-amylase that can be used in the present technique is an enzyme that acts on starch and mainly hydrolyzes α-1,4 glycosidic bonds. The α-amylase that can be used in the present technique may be an enzyme further having other functions as long as the α-amylase activity is its main activity.

The origin of α-amylase that can be used in the present technique is not particularly limited, and examples thereof include α-amylases derived from organisms of the genus Aspergillus (e.g., Aspergillus oryzae, Aspergillus niger, etc.) and the genus Bacillus (e.g., Bacillus amyloliquefaciens, Bacillus subtilis, Bacillus licheniformis, etc.), preferably α-amylases derived from the genus Bacillus or α-amylases derived from Aspergillus, more preferably α-amylase derived from Bacillus amyloliquefaciens or α-amylase derived from Bacillus licheniformis.

The α-amylase that can be used in the present technique can be prepared from the culture solution of the microorganism from which the above-mentioned α-amylase is derived. Specifically, the α-amylase can be easily prepared by culturing an α-amylase-producing bacterium and isolating the α-amylase using known means, or by using a gene recombination technique, and the like.

In the present technique, commercially available α-amylases can also be used, and a preferred example of the commercially available α-amylase is α-amylase (derived from Bacillus amyloliquefaciens) manufactured by Amano Enzyme Inc.

The amount of α-amylase added in the cyclodextrin-producing enzyme and α-amylase action step can be freely set as long as it does not impair the effects of the present technique. The specific lower limit of the amount of α-amylase added in the present technique can be set to, for example, 0.05 U or more per 1 g of a plant-based raw material, and from the viewpoint of further enhancing the effect of increasing the cyclodextrin content, it can be set to preferably 0.1 U or more, more preferably 0.5 U or more.

Furthermore, the lower limit of the amount of α-amylase added can be set to, for example, 0.1 U or more per 1 g of a polysaccharide (preferably starch) used in the production of a plant-based food and beverage, and from the viewpoint of further enhancing the effect of increasing the cyclodextrin content, it can be set to preferably 0.2 U or more, more preferably 1 U or more.

The upper limit of the amount of α-amylase added is not particularly limited as long as it does not impair the effects of the present technique, but can be set to, for example, 1,000 U or less, 200 U or less, 100 U or less, preferably 50 U or less, more preferably 10 U or less, even more preferably 5 U or less, 3 U or less, or 1 U or less per 1 g of a plant-based raw material.

In addition, the upper limit of the α-amylase content can be set to, for example, 2,000 U or less, 400 U or less, 200 U or less, preferably 100 U or less, more preferably 20 U or less, even more preferably 10 U or less, or 4 U or less per 1 g of a polysaccharide (preferably starch) used in the production of a plant-based food and beverage.

In the present technique, the α-amylase activity is a value measured by the measurement method described in the Examples below.

For the addition amount of a cyclodextrin-producing enzyme when a cyclodextrin-producing enzyme is further added in the cyclodextrin-producing enzyme and α-amylase action step, it is preferable that the sum of the addition amount in the cyclodextrin-producing enzyme action step and the addition amount in the cyclodextrin-producing enzyme and α-amylase action step is the specific addition amount of the cyclodextrin-producing enzyme described in the description of the cyclodextrin-producing enzyme action step described above.

Various conditions for the cyclodextrin-producing enzyme and α-amylase action step can be freely set as long as they do not impair the effects of the present technique. For example, the pH, temperature, action time, etc. can be set according to the physicochemical properties such as the optimum pH, stable pH range, optimum temperature, and temperature stability of the cyclodextrin-producing enzyme and α-amylase used. The pH can be set to, for example, pH 5.0 to 8.0, preferably pH 5.5 to 7.5, and more preferably pH 6.0 to 7.0. The temperature can be set to, for example, 30°C to 90°C, preferably 40°C to 85°C, and more preferably 50°C to 80°C. The action time can be set to, for example, 5 minutes to 12 hours, preferably 10 minutes to 6 hours, and more preferably 20 minutes to 150 minutes. The optimal reaction conditions can be determined through preliminary experiments.

(4) Recovery step

The recovery step is a step of recovering the plant-based food and beverage produced through the cyclodextrin-producing enzyme action step and the cyclodextrin-producing enzyme and α-amylase action step. As for the specific recovery method, one type or two or more types of general recovery methods in the production of a plant-based food and beverage can be freely combined and used depending on the type of the plant-based food and beverage to be produced.

The plant-based food and beverage produced through the cyclodextrin-producing enzyme action step, and the cyclodextrin-producing enzyme and α-amylase action step, is characterized by an increased cyclodextrin content. Specifically, for example, the cyclodextrin content in the plant-based food and beverage produced through the cyclodextrin-producing enzyme action step, and the cyclodextrin-producing enzyme and $\alpha$-amylase action step is 3.0 mg or more, preferably 3.5 mg or more, and more preferably 4.0 mg or more per 1 mL of the plant-based food and beverage. That is, the present technique can perform a recovery step of recovering a plant-based food and beverage having a cyclodextrin content of 3.0 mg or more, preferably 3.5 mg or more, more preferably 4.0 mg or more, and even more preferably 5.0 mg or more per 1 mL of the plant-based food and beverage.

One embodiment of the method for producing a plant-based food and beverage and the method for increasing the cyclodextrin content in a plant-based food and beverage according to the present technique includes the following steps (1) to (3). Note that after step (3), a step (4) of inactivating the enzyme and/or a step (5) of recovering the plant-based food and beverage with an increased cyclodextrin content may be added.

(1) A step of preparing a raw material containing plant-based carbohydrates
(2) A step of treating the prepared raw material with a cyclodextrin-producing enzyme
(3) A step of treating the raw material treated with the cyclodextrin-producing enzyme with the cyclodextrin-producing enzyme and $\alpha$-amylase

One embodiment of the method for producing a plant-based food and beverage and the method for increasing the cyclodextrin content in a plant-based food and beverage according to the present technique includes the following steps (1) to (3). Note that after step (3), a step (4) of inactivating the enzyme and/or a step (5) of recovering the plant-based food and beverage with an increased cyclodextrin content may be added.

(1) A step of preparing a raw material containing plant-based carbohydrates
(2) A step of treating the prepared raw material with a cyclodextrin-producing enzyme
(3) A step of adding $\alpha$-amylase to the raw material being treated with the cyclodextrin-producing enzyme while continuing the step (2), thereby treating the raw material with the cyclodextrin-producing enzyme and $\alpha$-amylase.

In the step (5), a plant-based food and beverage having a cyclodextrin content of 3.0 mg or more per 1 mL of the plant-based food and beverage can also be recovered.

2. Agent for increasing cyclodextrin content in plant-based food and beverage

The agent for increasing the cyclodextrin content in plant-based foods and beverages according to the present technique includes a first agent containing a cyclodextrin-producing enzyme and a second agent containing $\alpha$-amylase. For example, by adding the first agent to a plant-based raw material used in the production of a plant-based food and beverage to treat with the cyclodextrin-producing enzyme and then adding the second agent without inactivating the cyclodextrin-producing enzyme, the cyclodextrin-producing enzyme and $\alpha$-amylase action step can be allowed to proceed, and as described above, the cyclodextrin content in the plant-based food and beverage produced can be increased. The composition of the cyclodextrin content increasing agent will be described in detail below.

(1) First agent

The first agent contains a cyclodextrin-producing enzyme. The first agent may contain only the cyclodextrin-producing enzyme alone. That is, the first agent may be the cyclodextrin-producing enzyme itself. In addition, the first agent may contain one or more selected types of other enzymes that can be used in the production of a plant-based food and beverage, as long as the amount of cyclodextrin produced is not affected. Note that the details of the cyclodextrin-producing enzyme are the same as those of the cyclodextrin-producing enzyme described in the above-mentioned method for producing a plant-based food and beverage and the method for increasing the cyclodextrin content in a plant-based food and beverage, and therefore will not be described here.

The content of a cyclodextrin-producing enzyme in the first agent can be freely set as long as it does not impair the effects of the present technique. The content of a cyclodextrin-producing enzyme is the same as the amount of the cyclodextrin-producing enzyme added in the above-mentioned cyclodextrin-producing enzyme action step, so a detailed description of the content is omitted here.

(2) Second agent

The second agent contains $\alpha$-amylase. The second agent may contain only $\alpha$-amylase alone. That is, the second agent may be $\alpha$-amylase itself. The second agent may contain a cyclodextrin-producing enzyme in addition to $\alpha$-amylase. Furthermore, the second agent may contain one or more selected types of other enzymes that can be used in the production of a plant-based food and beverage, as long as the amount of cyclodextrin produced is not affected. Details of $\alpha$-

amylase are the same as those of $\alpha$-amylase described in the above-mentioned method for producing a plant-based food and beverage and method for increasing the cyclodextrin content in a plant-based food and beverage, and therefore will not be described here.

The content of $\alpha$-amylase in the second agent can be freely set as long as it does not impair the effect of the present technique. The content of $\alpha$-amylase is the same as the amount of $\alpha$-amylase added in the above-mentioned cyclodextrin-producing enzyme and $\alpha$-amylase action step, so a description is omitted here.

(3) Other

The first and second agents of the cyclodextrin content increasing agent for plant-based foods and beverages according to the present technique may contain one or more types of other components selected freely, as long as the effect of the present technique is not impaired. As other components, for example, components such as excipients, pH adjusters, colorants, flavoring agents, disintegrants, lubricants, and stabilizers that are usually used in formulation can be used. Furthermore, components with known or future functions may be used in combination as appropriate depending on the purpose.

In addition, the agent for increasing the cyclodextrin content in a plant-based food and beverage according to the present technique may further contain a third agent, a fourth agent, etc. that contain selected one or more types of other enzymes that can be used in the production of a plant-based food and beverage, or components having known or future functions, in addition to the first and second agents, as long as the amount of cyclodextrin produced is not affected.

3. Plant-based food and beverage

The plant-based food and beverage according to the present technique is a plant-based food and beverage produced using the above-mentioned cyclodextrin content increasing agent. A specific example of the plant-based beverage is oat beverage, more specifically oat milk (also called "oats milk"). A specific example of the plant-based food is plant-based yogurt.

The plant-based food and beverage according to the present technique has an increased amount of cyclodextrin, and the increased amount of cyclodextrin can mask off-flavors. The amount of cyclodextrin contained in the plant-based food and beverage according to the present technique is also not particularly limited. The amount of cyclodextrin contained in the plant-based food and beverage is, for example, 3.0 mg or more, preferably 3.5 mg or more, more preferably 4.0 mg or more, and even more preferably 4.5 mg or more, as the total amount of $\alpha$-cyclodextrin, per 1 mL of the plant-based food and beverage.

(EXAMPLES)

The present invention will be described in more detail below with reference to examples. Note that the examples described below are representative examples of the present invention, and the scope of the present invention should not be construed as being narrowed by these examples.

1. Enzyme used

Cyclodextrin-producing enzyme (CGT): cyclodextrin glucanotransferase derived from Anoxybacillus caldiproteolyticus (previously classified as Geobacillus stearothermophilus), manufactured by Amano Enzyme Inc.

$\alpha$-Amylase (AMY): $\alpha$-amylase derived from Bacillus amyloliquefaciens, manufactured by Amano Enzyme Inc.

2. Enzyme activity measurement method

[Method for measuring cyclodextrin-producing enzyme activity]

The activity of a cyclodextrin-producing enzyme can be measured by a method based on the cyclodextrin-producing enzyme activity test method in the 9th edition of the Japan's Specifications and Standards for Food Additives.

20 mL of water was added to 1.0 g of potato starch, and 5 mL of a sodium hydroxide test solution (1 mol/L) was gradually added while stirring to form a sticky paste. After heating in a boiling water bath for 3 minutes while stirring, 25 mL of water was added, cooled with running water, and the pH was adjusted to 5.5 with an acetic acid test solution (1 mol/L), and water was further added to make 100 mL to prepare a substrate solution. 10 mL of the substrate solution was measured and heated at 40°C for 10 minutes, and 1 mL of the enzyme solution was added and mixed. After incubating at 40°C for 10 minutes, the reaction was stopped by adding 10 mL of a hydrochloric acid test solution (0.1 mol/L). 10 mL of an iodine/potassium iodide test solution (0.4 mmol/L) was added to 1 mL of this reaction solution and mixed to prepare a subject solution. The iodine/potassium iodide test solution was prepared by dissolving 10.0 g of potassium iodide and 1.0 g of iodine in water to make 100 mL, and then diluting 200 fold with water. A comparative solution was prepared by using water instead of the reaction solution, and the absorbance at 660 nm of the subject solution and the comparative solution was measured. The amount of enzyme that reduces the blue iodine coloration of starch by 1% in 1 minute was defined as 1 unit (U).

[Method for measuring α-amylase activity]

10 mL of a 1% potato starch substrate solution (0.1 mol/L acetic acid (pH 5.0)) was heated at 37°C for 10 minutes, after which 1 mL of a sample solution containing α-amylase was added and immediately shaken. This solution was left to stand at 37°C for 10 minutes, after which 1 mL of this solution was added to 10 mL of a 0.1 mol/L hydrochloric acid test solution and immediately shaken. Next, 0.5 mL of this solution was measured, and 10 mL of a 0.0002 mol/L iodine test solution (Japanese Pharmacopoeia) was added and shaken, after which the absorbance (AT) at a wavelength of 660 nm was measured using water as a control. Separately, 1 mL of water was added instead of the sample solution, and the procedure was repeated to measure the absorbance (AB). The 0.0002 mol/L iodine test solution (Japanese Pharmacopoeia) was prepared by adding 10 mL of water to 12.7 g of iodine and 25 g of potassium iodide, mixing thoroughly, adding water to make 100 mL, and then diluting 2,500 fold with water. The α-amylase activity was calculated using the following formula. The amount of enzyme that reduces the color of potato starch caused by iodine by 10% in 1 minute is defined as 1 unit (1 U).

$$\text{α-Amylase activity (U/g, U/mL)} = \{(AB\text{-}AT)/AB\} \times 1/W$$

AT: absorbance of reaction solution
AB: absorbance of blank solution
W: Amount of sample in 1 mL of sample solution (g or mL)

3. Experimental example

(1) Experimental method

12.5 g of oat flour (PREMIUM OAT FLOUR, carbohydrate content 67.7 g/100 g, manufactured by Slow Food Kitchen) was suspended in 100 mL of water added, and a cyclodextrin-producing enzyme (CGT) was added thereto in an amount shown in Table 1 relative to the raw material. After the enzyme was added, the enzyme was allowed to act for 0 to 60 minutes while stirring in a 60°C water bath. Next, α-amylase (AMY) was added to the cyclodextrin-producing enzyme reaction solution in an amount shown in Table 1 relative to the raw material, and the enzyme was allowed to react for 30 to 120 minutes while stirring in a 60°C water bath. After the reaction, the enzyme was inactivated by treatment at 95°C for 10 minutes. For the quantitative determination of α-cyclodextrin, 1% glucoamylase was added after the enzyme inactivation treatment, and the enzyme was allowed to act at 60°C for 2 hours, and then treated at 95°C for 10 minutes to inactivate the enzyme. 190 μL of 1 mol/L trichloroacetic acid was added to 1.5 mL of the enzyme-inactivated oat solution, and the mixture was left to stand at room temperature for 10 minutes. After 10 minutes, the mixture was centrifuged (12,000 rpm, 10 minutes) to collect the supernatant. The collected supernatant was suspended in an equal amount of diethyl ether added. The mixture was centrifuged (12,000 rpm, 10 minutes), the organic solvent layer was discarded, and the aqueous layer was collected. This procedure was repeated twice. The collected aqueous layer was added with an equal amount of 99.5% ethanol (for high-performance liquid chromatography, manufactured by FUJIFILM Wako Pure Chemical Corp.), stirred, and filtered through a membrane filter, and the resulting mixture was subjected to analysis. High-performance liquid chromatography was used for the analysis, and the column used was MCL GEL CK04S manufactured by Mitsubishi Chemical Corporation. The amount of α-cyclodextrin produced was quantified from the peak area. In addition, the viscosity of a plant-based food and beverage was evaluated as "+" if the viscosity was clearly higher than that of water when stirred with a spatula, and "-" if the viscosity was close to that of water.

(2) Result

The results are shown in Table 1 below.

[Table 1]

|  | Control | Sample | | | Control | Sample | | Control | Sample | | Control | Sample | |
|---|---|---|---|---|---|---|---|---|---|---|---|---|---|
|  | 1 | 1-1 | 1-2 | 1-3 | 2 | 2-1 | 2-2 | 3 | 3-1 | 3-2 | 4 | 4-1 | 4-2 |
| Addition amount (U/g-raw material) of CGT | 2 | 2 | 2 | 2 | 2 | 2 | 2 | 2 | 2 | 2 | 2 | 2 | 2 |
| Addition amount (U/g-raw material) of AMY | 0 | 0.6 | 0.8 | 2.4 | 0 | 0.6 | 2.4 | 0 | 0.6 | 0.8 | 0 | 0.6 | 0.8 |
| CGT treatment time (min) | 0 | | | | 0 | | | 30 | | | 60 | | |
| CGT+AMY treatment time (min) | 60 | | | | 120 | | | 30 | | | 60 | | |
| Total treatment time (min) | 60 | | | | 120 | | | 60 | | | 120 | | |
| Evaluation | | | | | | | | | | | | | |
| Amount of $\alpha$-cyclodextrin (mg/mL) | 3.0 | 2.9 | 2.6 | 1.5 | 4.6 | 3.2 | 1.3 | 4.5 | 4.4 | 4.4 | 5.3 | 5.3 | 5.2 |
| Amount of $\alpha$-cyclodextrin (ratio relative to control) | 1.0 | 1.0 | 0.9 | 0.5 | 1.0 | 0.7 | 0.3 | 1.0 | 1.0 | 1.0 | 1.0 | 1.0 | 1.0 |
| Viscosity (appearance) | + | - | - | - | + | - | - | + | - | - | + | - | - |

(3) Observation

As shown in Samples 1-1 to 1-3, 2-1, and 2-2 in Table 1, the combined use of a cyclodextrin-producing enzyme and $\alpha$-amylase was able to sufficiently promote liquefaction and reduce viscosity, but the $\alpha$-cyclodextrin content in the plant-based food and beverage decreased in proportion to the amount of $\alpha$-amylase added. On the other hand, as shown in Samples 3-1, 3-2, 4-1, and 4-2 in Table 1, it was confirmed that by treating the plant-based raw material with only the cyclodextrin-producing enzyme before treatment with the cyclodextrin-producing enzyme and $\alpha$- amylase, the decrease in the $\alpha$-cyclodextrin content was significantly suppressed, and despite sufficient liquefaction, the $\alpha$-cyclodextrin content was increased to the same extent as in the case where $\alpha$-amylase was not added.

**Claims**

1. A method for producing a plant-based food and beverage comprising

    a cyclodextrin-producing enzyme action step in which a cyclodextrin-producing enzyme is allowed to act on a plant-based raw material; and
    a cyclodextrin-producing enzyme and $\alpha$-amylase action step in which a cyclodextrin-producing enzyme and $\alpha$-amylase are allowed to act thereon after the cyclodextrin-producing enzyme action step.

2. A method for increasing the cyclodextrin content in a plant-based food and beverage, comprising

    a cyclodextrin-producing enzyme action step in which a cyclodextrin-producing enzyme is allowed to act on a plant-based raw material; and

a cyclodextrin-producing enzyme and $\alpha$-amylase action step in which a cyclodextrin-producing enzyme and $\alpha$-amylase are allowed to act thereon after the cyclodextrin-producing enzyme action step.

3. An agent for increasing the cyclodextrin content in a plant-based food and beverage comprising

a first agent containing a cyclodextrin-producing enzyme; and
a second agent containing $\alpha$-amylase.

4. A plant-based food and beverage, in which the agent for increasing the cyclodextrin content according to claim 3 is used.

## INTERNATIONAL SEARCH REPORT

| International application No. |
| --- |
| **PCT/JP2023/039815** |

### A. CLASSIFICATION OF SUBJECT MATTER

*A23L 5/00*(2016.01)i; *A23L 2/00*(2006.01)i; *A23L 2/52*(2006.01)i; *A23L 2/66*(2006.01)i; *A23L 7/104*(2016.01)i;
*C12N 9/10*(2006.01)i; *C12N 9/28*(2006.01)i; *C12P 19/14*(2006.01)i; *C12P 19/18*(2006.01)i
FI:  A23L5/00 J; A23L2/00 B; A23L2/52; A23L2/66; A23L5/00 N; A23L7/104; C12N9/10; C12N9/28; C12P19/14 Z;
C12P19/18

According to International Patent Classification (IPC) or to both national classification and IPC

### B. FIELDS SEARCHED

Minimum documentation searched (classification system followed by classification symbols)

A23L2/00-35/00; C12P19/14; C12P19/18; A23C11/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS/FSTA/AGRICOLA (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 2-255095 A (SANYO KOKUSAKU PULP CO LTD) 15 October 1990 (1990-10-15) claims, p. 2, upper right column, line 11 to lower left column, line 5, p. 4, upper right column, lines 3-9, table 1, examples 1, 3, 4 | 1-4 |
| X | JP 6-510435 A (AMERICAN MAIZE-PRODUCTS COMPANY) 24 November 1994 (1994-11-24) claims, p. 2, upper right column, lines 14-17, example 1 | 3-4 |
| X | JP 2001-327299 A (NIPPON SHOKUHIN KAKO CO LTD) 27 November 2001 (2001-11-27) claims 1-11, paragraph [0003], examples 1-4, 6, 8 | 3-4 |
| X | CN 111084322 A (ANHUI CHUNGU FOOD HEALTH TECHNOLOGY RESEARCH CO., LTD.) 01 May 2020 (2020-05-01) claims 1-6, paragraphs [0019]-[0025] | 4 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 January 2024** | **23 January 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2023/039815**

### C. DOCUMENTS CONSIDERED TO BE RELEVANT

| Category* | Citation of document, with indication, where appropriate, of the relevant passages | Relevant to claim No. |
|---|---|---|
| X | CN 103719702 A (WU, Zhuangzhi) 16 April 2014 (2014-04-16)<br>claims 1-10, examples 1-4 | 4 |

**INTERNATIONAL SEARCH REPORT**
Information on patent family members

International application No.

**PCT/JP2023/039815**

| Patent document cited in search report | | | Publication date (day/month/year) | Patent family member(s) | | | Publication date (day/month/year) |
|---|---|---|---|---|---|---|---|
| JP | 2-255095 | A | 15 October 1990 | (Family: none) | | | |
| JP | 6-510435 | A | 24 November 1994 | US | 5620872 | A | |
| | | | | claims, column 1, lines 19-23, example 1 | | | |
| | | | | WO | 1993/010255 | A1 | |
| | | | | EP | 672164 | A1 | |
| | | | | CN | 1072185 | A | |
| JP | 2001-327299 | A | 27 November 2001 | (Family: none) | | | |
| CN | 111084322 | A | 01 May 2020 | (Family: none) | | | |
| CN | 103719702 | A | 16 April 2014 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018075029 A
- JP HEI09262091 A